(19) Europäisches Patentamt / European Patent Office / Office européen des brevets

(11) **EP 1 709 952 B1**

(12) **EUROPEAN PATENT SPECIFICATION**

(45) Date of publication and mention
of the grant of the patent:
**24.10.2012 Bulletin 2012/43**

(51) Int Cl.:
*A61K 8/06* (2006.01)     *A61K 8/86* (2006.01)
*A61Q 19/10* (2006.01)

(21) Application number: **04819829.5**

(22) Date of filing: **30.11.2004**

(86) International application number:
**PCT/JP2004/017787**

(87) International publication number:
**WO 2005/053623 (16.06.2005 Gazette 2005/24)**

(54) **COMPOSITION CONTAINING POLYGLYCEROL/MEDIUM-CHAIN FATTY ACID ESTER**

ZUSAMMENSETZUNG MIT POLYGLYCEROL/MITTELKETTIGEN FETTSÄUREESTER

COMPOSITION CONTENANT UN ESTER D'ACIDE GRAS A CHAINE MEDIANE/ POLYGLYCEROL

(84) Designated Contracting States:
**AT BE BG CH CY CZ DE DK EE ES FI FR GB GR HU IE IS IT LI LU MC NL PL PT RO SE SI SK TR**

(30) Priority: **04.12.2003 JP 2003405875**

(43) Date of publication of application:
**11.10.2006 Bulletin 2006/41**

(73) Proprietor: **TAIYO KAGAKU Co., LTD.**
**Yokkaichi-shi, Mie 5100825 (JP)**

(72) Inventors:
• **IWANAGA, Tetsuro**
**Mie 510-0825 (JP)**
• **UCHIDA, Kazuhito**
**Mie 510-0825 (JP)**
• **TAKEUCHI, Nobuyuki**
**Yokohama-shi**
**Kanagawa 244-0806 (JP)**

• **ABE, Yoshihisa**
**Yokohama-shi**
**Kanagawa 244-0806 (JP)**

(74) Representative: **HOFFMANN EITLE**
**Patent- und Rechtsanwälte**
**Arabellastrasse 4**
**81925 München (DE)**

(56) References cited:
**EP-A- 0 334 777      DE-A1- 4 226 174**
**JP-A- 6 192 065      JP-A- 8 099 852**
**JP-A- 2001 025 654      JP-A- 2003 012 456**
**JP-A- 2003 040 708      JP-A- 2003 238 396**
**JP-A- 2004 067 587      JP-A- 2004 277 375**
**US-A- 3 637 774      US-A- 5 397 497**

Remarks:
The file contains technical information submitted after the application was filed and not included in this specification

EP 1 709 952 B1

**Description**

TECHNICAL FIELD

The present invention relates to the use of a polyglycerol medium-chain fatty acid ester-containing composition for removing make-up.

BACKGROUND ART

In the field of cosmetics, cleansing cosmetics for the purpose of removing makeup are in the form of cream, milky lotion, liquid or the like. Also, the cleansing cosmetics can be classified by types into emulsion type, oil type and aqueous type, and have been utilized depending upon their respective uses. However, the aqueous type cleansing cosmetics containing no oil at all or a small amount of oil have disadvantage that the remover capability is weakened even though they give reduced oily feel after cleansing. Therefore, in recent years, the mainstream of the cleansing cosmetics have been a liquid oil-based makeup remover which has excellent affinity to makeup soil and is capable of easily washing off with water.

Makeup soil is removed using the makeup remover by first allowing a makeup remover to have affinity with makeup soil, thereby migrating the soil into an oily component, thereafter contacting the oily component with water to thereby form an oil-in-water emulsion of the soil-containing oily component, and directly washing off the emulsion. However, since the makeup remover is self-emulsifiable oil solution comprising a mixed system of an oily component and a surfactant, or a reversed micellar oil solution containing a small amount of water and the oil solution cannot solubilize a large amount of water has a small amount of water solubilized, phase separation take place when the amount of water exceeds the limit of solubilization, thereby forming white turbid emulsion. Therefore, an emulsion is formed spontaneously by the contact of water, so that remover capability or feel of use is worsened in a state where the skin is wet, so that its use in a bathroom is especially unsuitable.

The microemulsion is a system in which an oil (or water) is solubilized in an amount larger than a usual micellar solution (or reverse micellar oil solution), and its external appearance is an isotropic solution showing transparent to bluish color. In the microemulsion, there are oil-in-water type (O/W) and water-in-oil type (W/O) in the same manner as the usual macroemulsion. Furthermore, there is a bicontinuous-type microemulsion in which both an oil phase and a water phase are continuous in which large amounts of water and oil are solubilized.

It has been known that the microemulsion is obtained by adding a medium-chain alcohol having 5 to 10 carbon atoms to three components of water/surfactant/oil (see Non-Patent Publication 1). However, the medium-chain alcohol as described above is difficult to be utilized in the use of cosmetics and medicaments from the viewpoint of skin irritation or the like.

Non-Patent Publication 1: J. H. Schulman, W. Stoeckenius, L. M. Prince, J. Phys. Chem., 63, 1677 (1959)

EP 0582246 A2 relates to a mixture of polyglycerol fatty acid esters composed of polyglycerol mono-, di- and polyesters having a degree of polymerisation of 2 to 8 and of at least one saturated and/or unsaturated fatty acid, wherein the fatty acid component is composed of one or more fatty acids selected from saturated and/or unsaturated, branched and/or unbranched C6- to C 14-fatty acids and contains less than 10% by weight of fatty acids having more than 14 C atoms. The composition is used in the preparation of bath additives.

JP 8099852 A relates to a hircismus preventive which contains a middle-size fatty acid ester which can be obtained by esterification of an 8-14 C fatty acid such as palmitic or lauric acid with a polyhydric alcohol such as diglycerol or triglycerol. This preventive is preferably prepared in a cream of water-in-oil emulsion wherein the amount of water is preferably 10-60%.

JP 2003-012456 A relates to a cleansing preparation includes (A) a polyglycerol fatty acid ester ≥8.0 in HLB number, an ester of a polyglyderol ≥3 in average polymerization degree and at least one fatty acid selected from 8-22C saturated or unsaturated fatty acids and (B) a maltitol hydroxyaliphatic ether and/or alkylglucoside-based surfactant; wherein the compounding amounts of the components A and B are 1.0-25.0 wt.% and 0.5-15.0 wt.% respectively.

JP 2001-025654 A relates to a micro-emulsion which is produced using a surfactant containing an aliphatic acid polyglycerin ester and an aliphatic acid sucrose diester. The emulsion is said to be used for cosmetics such as a cleaning agents, medicines such as an oral administration drugs, and foods such as ice cream.

JP 2003-238396 A relates to an emulsified composition containing the coenzyme Q10 in the high concentration. The composition comprises the coenzyme Q10, an oil phase component, a polyhydric alcohol and an emulsifier to prevent crystallization after the emulsification.

JP 2004-067587 A relates to a cosmetic capable of inhibiting the proliferation of microbes living on the skin of the human body and which is low in skin irritancy. The cosmetic contains an antimicrobial agent composition comprising (A) ε-polylysine and/or a salt thereof, (B) an electrolyte having pH buffering ability and (C) an amino acid.

JP 2004-277375 A relates to a method for producing a clear and transparent liquid composition in which particle size distribution of micelle particle is sharp which comprises mixing a fat-soluble medicine with a polyglycerol fatty acid ester, a polyoxyethylene-based nonionic surfactant, a water-soluble polyhydric alcohol and water.

JP 2003-040708 A relates to a microorganism-controlling composition which contains the primary ester and the secondary ester of a polyglycerol in an effective molar ratio of 8:1-25:1.

EP 0334777 A1 relates to a microemulsion comprising a mixture of at least one water-soluble phase, at least one lipid phase, at least one surfactant and at least one cosurfactant, wherein the surfactant is a mixed ester of glycerol and polyethylene glycol and represents at least ten per cent (10%) by weight of the mixture, the cosurfactant is an ester of polyglycerol and a liquid fatty acid, which is in the mixture in a smaller proportion than that of the surfactant and represents at least five per cent (5%) by weight of the mixture, and the lipid compound possesses a hydrophilic/lipophilic balance not exceeding three.

## DISCLOSURE OF THE INVENTION

## PROBLEMS TO BE SOLVED BY THE INVENTION

The present inventors have found that a composition comprising a specified polyglycerol medium-chain fatty acid ester as a co-surfactant in addition to a nonionic surfactant forms a microemulsion capable of solubilizing a large amount of water, and has excellent water dispersiblity and self-emulsifiability in a large amount of water. The present invention has been accomplished thereby.

An object of the present invention is to provide a polyglycerol medium-chain fatty acid ester-containing composition capable of forming a water-in-oil microemulsion, the microemulsion being capable of solubilizing a large amount of water, and the composition having excellent dispersibility and self-emulsifiability in water and being usable for removing make-up.

## MEANS TO SOLVE THE PROBLEMS

The present invention relates to the use of a polyglycerol medium-chain fatty acid ester-containing composition for removing make-up, comprising a polyglycerol medium-chain fatty acid ester formed by esterification of a medium-chain fatty acid having 6 to 10 carbon atoms and a polyglycerol having an average degree of polymerization of 3 or more and less than 100, a nonionic, and an oil agent having a melting point at 25°C or lower, or having fluidity in which solid substances are dispersed in a liquid substance having a melting point of less than 25°C, wherein the oil agent is present in an amont of 70 to 98% by weight based on the polyglycerol medium-chain fatty acid ester-containing composition.

## EFFECTS OF THE INVENTION

The polyglycerol medium-chain fatty acid ester-containing composition for the use of the present invention is capable of forming a water-in-oil microemulsion, the microemulsion being capable of solubilizing a large amount of water, and the composition having excellent dispersibility and self-emulsifiability in water. Therefore, the composition according to the present invention,when used as cleaning cosmetics, provides affinity to makeup soil and its remover capability is not impaired even when the skin is wet, so that the composition is excellent for removing make-up.

## BEST MODE FOR CARRYING OUT THE INVENTION

The polyglycerol medium-chain fatty acid ester-containing composition for the use of the present invention comprises at least a polyglycerol medium-chain fatty acid ester formed by esterification of a medium-chain fatty acid having a specified number of carbon atoms and a polyglycerol having a specified average degree of polymerization, a nonionic surfactant and an oil agent.

The medium-chain fatty acid has a number of carbon atoms of from 6 to 10, preferably from 8 to 10, from the viewpoint of low-temperature stability. Therefore, specific examples of preferred medium-chain fatty acids include caprylic acid, capric acid and the like.

The polyglycerol has an average degree of polymerization of from 3 or more and less than 100, preferably from 3 to 50, from the viewpoint of usefulness.

The content of the cyclic compound in the polyglycerol is preferably 25% or less, more preferably 20% or less. The content of the cyclic compound can be analyzed with liquid chromatography-mass spectrometer (LC/MS).

The polyglycerol medium-chain fatty acid ester has an HLB of preferably from 7 to 15, more preferably from 8 to 13, from the viewpoint of compatibility with an oil agent and dispersibility in water. Here, HLB number is calculated by the formula:

$$HLB = 20 \ (1 - S/A)$$

wherein S is a saponification value of the ester, and A is a neutralization value of the fatty acid.

The polyglycerol medium-chain fatty acid ester shows a transparent liquid state or a pasty state, and is not solidified even at a low temperature (0°C).

The polyglycerol medium-chain fatty acid ester can be easily obtained by esterification of a medium-chain fatty acid and a polyglycerol by a conventional method.

The polyglycerol medium-chain fatty acid ester may be used alone, or as a mixture of two or more kinds thereof having different degrees of polymerization of the polyglycerol or different degrees of esterification.

The content of the polyglycerol medium-chain fatty acid ester, for instance, in the case where the polyglycerol medium-chain fatty acid ester is used for cleansing cosmetics, is preferably from 0.1 to 80% by weight, more preferably from 1 to 50% by weight, especially preferably from 2 to 30% of the polyglycerol medium-chain fatty acid ester-containing composition, from the viewpoint of affinity to makeup soil.

The nonionic surfactant includes polyoxyethylene alkyl ethers, glycerol fatty acid esters, polyglycerol fatty acid esters (except for the above-mentioned polyglycerol medium-chain fatty acid ester), polyalkylene glycol fatty acid esters, sorbitan fatty acid esters, sugar fatty acid esters, pentaerythritol fatty acid esters, fatty acid alkanolamides, ethers formed between a polyoxyalkylene glycol and a monohydric or polyhydric alcohol, polyoxyalkylene sugar ethers, condensates formed between a fatty amine and a polyoxyalkylene glycol, alkyl or alkenyl polyglycosides, and the like.

Among these nonionic surfactants, a nonionic surfactant being highly safe and showing a liquid state or a pasty state at 25°C is desirable, and one having a polyglycerol moiety having an average degree of polymerization of from 2 to 30 or a polyoxyalkylene group having an alkylene moiety with 2 to 4 carbon atoms, and an average number of moles added of from 1 to 80 is preferable.

Therefore, specific examples of preferred nonionic surfactants include polyglycerol(average degree of polymerization: 3 to 15) fatty acid(number of carbon atoms: 12 to 24) esters, polyoxyethylene(average number of moles added: 3 to 40) alkyl(number of carbon atoms: 12 to 24) ethers, polyoxyethylene(average number of moles added: 3 to 40) fatty acid(number of carbon atoms: 12 to 24) esters, polyoxyethylene(average number of moles added: 3 to 40) glycerol fatty acid(number of carbon atoms: 12 to 24) esters, polyoxyethylene(average number of moles added: 3 to 40) hardened castor oils, polyoxyethylene(average number of moles added: 3 to 40) alkyl ether polyglycosides, fatty acid(number of carbon atoms: 12 to 24) polyoxyethylene(average number of moles added: 3 to 40) sorbitan esters, and the like.

The nonionic surfactant may be used alone or as a mixture of two or more kinds thereof having different HLBs or the like.

The content of the nonionic surfactant, for instance, in the case where the nonionic surfactant is used for cleansing cosmetics, is preferably from 0.1 to 80% by weight, more preferably from 1 to 40% by weight, especially preferably from 2 to 30% by weight, of the polyglycerol medium-chain fatty acid ester-containing composition, from the viewpoint of remover capability and rinsability.

The polyglycerol medium-chain fatty acid ester-containing compositin for the use of the present invention further comprises an oil agent. The oil agent shows a liquid or pasty state at 25°C. In the present invention, the phrase "shows (showing) a liquid or pasty state at 25°C" refers to one having a melting point of 25°C or lower, or one having fluidity in which solid substances are dispersed in a liquid substance having a melting point of less than 25°C.

The oil agent includes natural animal or plant fats and oils and semi-synthesized fats and oils, hydrocarbon oils, ester oils, glyceride oils, silicone oils, fat-soluble vitamins, higher fatty acids, components of purified oils from animals and plants or synthetic oils, and the like.

The natural animal or plant fats and oils and semi-synthesized fats and oils include avocado oil, linseed oil, almond oil, olive oil, wheat germ oil, sesame oil, rice germ oil, rice bran oil, safflower oil, soybean oil, evening primrose oil, Indian corn oil, rapeseed oil, horse fat, palm oil, palm kernel oil, castor oil, sunflower oil, jojoba oil, macadamia nut oil, coconut oil, hardened coconut oil, peanut oil, lanolin and the like.

The hydrocarbon oil includes squalane, squalene, liquid paraffin, Vaseline and the like.

The ester oil includes diisobutyl adipate, 2-hexyldecyl adipate, di-2-heptylundecyl adipate, isostearyl isostearate, trimethylolpropane triisostearate, cetyl 2-ethylhexanoate, neopentyl glycol di-2-ethylhexanoate, trimethylolpropane tri-2-ethylhexanoate, pentaerythritol tetra-2-ethylhexanoate, cetyl octanoate, oleyl oleate, octyl dodecyl oleate, decyl oleate, neopentyl glycol dicaprate, 2-ethylhexyl succinate, isocetyl stearate, butyl stearate, diisopropyl sebacate, cetyl lactate, tetradecyl lactate, isopropyl myristate, octyl palmitate, 2-ethylhexyl palmitate, 2-hexyldecyl palmitate, 2-heptylundecyl palmitate, cholesteryl 12-hydroxystearate, phytostearyl oleate, diisostearyl malate, paramethoxycinnamic acid ester, pentaerythritol tetrarosinate, and the like.

The glyceride oil includes glyceryl triisostearate, glyceryl triisopalmitate, glyceryl tri-2-ethylhexanoate, glycerol tritetradecanoate, glyceryl diparamethoxycinnamate monoisoocylate, and the like.

The silicone oil includes higher alkoxy-modified silicones, alkyl-modified silicones and higher fatty acid ester-modified silicones such as dimethyl polysiloxane, methylphenyl polysiloxane, methyl hydrogen polysiloxane, octamethyl cyclopentanesiloxane, decamethyl cyclohexasiloxane, and stearoxy silicone; and the like.

The fat-soluble vitamin includes tocopherol or a derivative thereof, retinol or a derivative thereof, and the like.

The content of the oil agent, from 70 to 98% by weight, of the polyglycerol medium-chain fatty acid ester-containing composition, from the viewpoint of remover capability and rinsability.

Since the polyglycerol medium-chain fatty acid ester-containing composition of the present invention can form a microemulsion capable of solubilizing a large amount of water, the composition may be not only a non-aqueous composition which contains no water but also an aqueous composition containing water.

The weight ratio of the polyglycerol medium-chain fatty acid ester to the oil agent (polyglycerol medium-chain fatty acid ester/oil agent) is preferably from 10/0.1 to 0.1/10, from the viewpoint of preventing phase separation by containment of water.

The polyglycerol medium-chain fatty acid ester-containing composition for the use of the present invention may further contain an oily gelation agent, a lower alcohol, a powder, functional beads, a capsule, an antioxidant, an ultraviolet absorbent, a plant extract, a moisturizing agent, a bactericidal agent, an anti-inflammatory agent, a preservative, a pigment, a perfume or the like.

The oily gelation agent includes organic modified bentonite, hydrophobic silicic acid, silicic acid anhydride, starch fatty acid esters, and the like. The oily gelation agent is useful in the adjustment of viscosity. For instance, the properties of the composition of the present invention can be properly adjusted to a state of transparent to semi-transparent liquid to viscous paste.

The polyglycerol middle-chain fatty acid ester-containing composition for the use of the present invention can form a water-in-oil type microemulsion capable of solubilizing a large amount of water. Also, in the process of adding water, since the interfacial tension between oil and water phases is reduced by the formation of the microemulsion, the microemulsion is easily emulsified by further adding a large amount of water to form fine emulsion, the composition also has excellent water dispersibility and self-emulsifiability in water. Furthermore, since the polyglycerol is prepared by using glycerol as a starting raw material, the resulting composition has low skin irritation, and also excellent properties in the aspect of safety.

Specifically, cleansing cosmetics comprising the polyglycerol medium-chain fatty acid ester-containing composition for the use of the present invention have excellent affinity to makeup soil, and have remarkably excellent remover capability as compared to those of conventional oil-based makeup removers in the cleansing step, without losing their remover capability even in a wet skin state.

The content of the polyglycerol medium-chain fatty acid ester-containing composition in the cosmetics can be properly selected depending upon the manufactured article used and their purposes, and is not particular limited. For cleansing cosmetics, it is preferable that the content of the polyglycerol medium-chain fatty acid ester-containing composition is adjusted so that the content of the polyglycerol medium-chain fatty acid ester is preferably from 0.1 to 80% by weight, more preferably from 1 to 50% by weight, even more preferably from 2 to 30% by weight, of the cosmetics, from the viewpoint of affinity to makeup soil.

The cosmetics can be prepared in the same manner as usual depending upon their purposes and the like except that the polyglycerol medium-chain fatty acid ester-containing composition for the use of the present invention is used. The time of adding or method or adding the polyglycerol medium-chain fatty acid ester-containing composition is not particularly limited as long as the exhibition of the desired effects of the present invention is obtained.

EXAMPLES

The present invention will be specifically described hereinbelow by the Examples, without intending to limit the scope of the present invention thereto.

Examples 1 to 9 and Comparative Examples 1 to 5

An oil-based makeup remover comprising a polyglycerol medium-chain fatty acid ester- containing composition was prepared by a conventional method using the raw materials shown in Table 1.

1. Evaluation for Feel of Use

Ten specialist panelists who were provided with makeup took on their hands 1 g of a makeup remover, and applied over an entire face with both hands and massaged for 30 seconds, and thereafter washed off with water. In the course of treatment, the affinity of makeup soil, rinsability and oily feel after washing were evaluated in 5 ranks from 1 (poor or being sensed) to 5 (excellent or not being sensed), and an average score of 10 individuals was calculated. The results are shown in Table 1 in accordance with the following evaluation criteria.

[Evaluation Criteria]

O: An average score of 4 or more;
Δ: An average score of 3 or more and less than 4; and
✕: An average score of less than 3.

2. Evaluation for Remover Capability (State Where Skin Was Not Being in Wet State)

A lipstick was applied to forearm in an area of 2 cm × 2 cm, and allowed to stand for 30 minutes. Thereafter, 0.5 of a makeup remover was applied thereto. Thereafter, the forearm was massaged for 30 seconds, so as to remove the lipstick therefrom, and washed off with water. The extent of removal of the lipstick was visually examined. The results are shown in Table 1 in accordance with the following evaluation criteria.

[Evaluation Criteria]

O: Excellently removed;
Δ: Not removed much; and
✕ : Not removed at all.

3. Evaluation for Remover Capability (State Where Skin Was Being in Wet State)

A lipstick was applied to forearm in an area of 2 cm × 2 cm, and allowed to stand for 30 minutes. The portion to which the lipstick was applied was wetted with water, and 0.5 of a makeup remover was applied thereto. Thereafter, the forearm was massaged for 30 seconds, so as to remove the lipstick therefrom, and washed off with water. The extent of removal of the lipstick was visually examined. The results are shown in Table 1 in accordance with the following evaluation criteria.

[Evaluation Criteria]

O: Excellently removed;
Δ: Not removed much; and
✕: Not removed at all.

4. Confirmation for Formation of Microemulsion

One gram of a makeup remover was weighed in a test tube, and 1 g of water was added thereto while stirring. The state was visually examined. In the case where a microemulsion was formed, the solution becomes transparent. The results are shown in Table 1 in accordance with the following evaluation criteria.

[Evaluation Criteria]

O: Transparent;
Δ: Semi-transparent; and
✕ : White turbid.

[Table 1]

| | Examples | | | | | | | | | Comparative Examples | | | | |
|---|---|---|---|---|---|---|---|---|---|---|---|---|---|---|
| | 1 | 2 | 3 | 4 | 5 | 6 | 7 * | 8 | 9* | 1 | 2 | 3 | 4 | 5 |
| **Polyelycerol Medium-Chain Fatty Acid Ester** | | | | | | | | | | | | | | |
| Pentadecaglycerol Pentacaprylate (HLB: 12) | | 10 | | 15 | 10 | | 5 | 10 | 10 | | | | | |
| Eicosaglycerol Hexacaprylate (HLB: 13) | 10 | | 10 | | | 10 | 5 | | | | | | | 20 |
| **Nonionic Surfactant** | | | | | | | | | | | | | | |
| Decaglycerol Monoisostearate | 20 | | | | | | | | | 20 | | | | |
| Decaglycerol Trilaurate | | 20 | | | | | | | 20 | | | | | |
| Decaglycerol Diisostearate | | | 10 | | | | 10 | | | | | 10 | | |
| Polyoxyethylene (10) Hardened Castor Oil* | | | | 15 | | | 10 | | | | 20 | 10 | | |
| Polyoxyethylene (20) Sorbitan Monooleate* | | | | | 20 | | | 5 | | | | | 20 | |
| Polyoxyethylene (10) Oleyl Ether* | | | | | | | 10 | 5 | | | | | | |
| **Oil Agent** | | | | | | | | | | | | | | |
| Octyl Palmitate | 50 | 50 | | 70 | 30 | 60 | 50 | | 20 | 60 | 80 | | 40 | |
| Liquid Paraffin | 10 | 10 | 10 | | 5 | 10 | | 20 | | 10 | | 10 | 5 | 10 |
| Isopropyl Myristate | | | 70 | | 30 | | 10 | 30 | 10 | | | 70 | 30 | 70 |
| Olive Oil | 10 | 10 | | | 5 | 10 | | 30 | | 10 | | | 5 | |
| Water | | | | | | | | | 40 | | | | | |
| **Feel of Use** | | | | | | | | | | | | | | |
| Affinity to Soil | ○ | ○ | ○ | ○ | ○ | ○ | ○ | ○ | ○ | △ | ○ | ○ | ○ | × |
| Rinsability | ○ | ○ | ○ | ○ | ○ | ○ | ○ | ○ | ○ | △ | ○ | ○ | ○ | × |
| Absence of Oily Feel | ○ | ○ | ○ | ○ | ○ | ○ | ○ | ○ | ○ | △ | ○ | △ | △ | × |
| **Remover Capability** | | | | | | | | | | | | | | |
| Skin not being in wet state | ○ | ○ | ○ | ○ | ○ | ○ | ○ | ○ | ○ | △ | ○ | ○ | △ | × |
| Skin being in wet state | ○ | ○ | ○ | ○ | ○ | ○ | ○ | ○ | ○ | × | × | × | × | × |

(continued)

| Remover Capability | | | | | | | | | | | | | |
|---|---|---|---|---|---|---|---|---|---|---|---|---|---|
| Formation of Microemulsion | ○ | ○ | ○ | ○ | ○ | ○ | ○ | ○ | ○ | × | × | × | × | × |

Note) The used amount is expressed by parts by weight.
*Number inside parenthesis shows an average number of moles of oxyethylene group added.
* Reference Examples

It can be seen from the above results that all of the oil-based makeup removers of the Examples in which the microemulsion was formed had excellent feel of use, and had excellent remover capability not only in the case where the skin was not in a wet state but also in a wet state, as compared to those of Comparative Examples.

INDUSTRIAL APPLICABILITY

The polyglycerol medium-chain fatty acid ester-containing composition can be suitably used for removing make-up

**Claims**

1. Use of a polyglycerol medium-chain fatty acid ester-containing composition for removing make-up, the composition comprising a polyglycerol medium-chain fatty acid ester formed by esterification of a medium-chain fatty acid having 6 to 10 carbon atoms and a polyglycerol having an average degree of polymerization of 3 or more and less than 100, a nonionic surfactant, and an oil agent having a melting point of 25 °C or lower, or having fluidity in which solid substances are dispersed in a liquid substance having a melting point of less than 25 °Cwherein the oil agent is present in an amount of 70 to 98% by weight based on the polyglycerol medium-chain fatty acid ester-containing composition.

2. The use according to claim 1, wherein the oil agent is selected from natural animal or plant fats and oils and semi-synthesized fats and oils, hydrocarbon oils, ester oils, glyceride oils, silicone oils, fat-soluble vitamins, higher fatty acids, and components of purified oils from animals and plants or synthetic oils.

**Patentansprüche**

1. Verwendung einer Zusammensetzung, die einen Polyglycerolester einer mittelkettigen Fettsäure enthält, zur Entfernung von Makeup, worin die Zusammensetzung einen Polyglycerolester einer mittelkettigen Fettsäure umfasst, der durch Veresterung einer mittelkettigen Fettsäure mit 6 bis 10 Kohlenstoffatomen und eines Polyglycerols mit einem mittleren Polymerisationsgrad von 3 oder mehr und weniger als 100 gebildet wird, ein nicht-ionisches Tensid und ein Ölmittel, das einen Schmelzpunkt von 25°C oder weniger oder ein Fließvermögen aufweist, in dem feste Substanzen in einer flüssigen Substanz mit einem Schmelzpunkt von weniger als 25°C dispergiert werden, umfasst, wobei das Ölmittel in einer Menge von 70 bis 98 Gew.%, bezogen auf die Zusammensetzung, die den Polyglycerolester der mittelkettigen Fettsäure enthält, vorliegt.

2. Verwendung gemäß Anspruch 1, worin das Ölmittel ausgewählt ist aus natürlichen tierischen oder pflanzlichen Fetten und Ölen und halbsynthetischen Fetten und Ölen, Kohlenwasserstoffölen, Esterölen, Glyceridölen, Silikonölen, fettlöslichen Vitaminen, höheren Fettsäuren und Bestandteilen von gereinigten Ölen aus Tieren oder Pflanzen oder synthetischen Ölen.

**Revendications**

1. Utilisation d'une composition contenant un ester d'acide gras à chaîne moyenne de polyglycérol pour enlever un maquillage, la composition comprenant un ester d'acide gras à chaîne moyenne de polyglycérol formé par estérification d'un acide gras à chaîne moyenne ayant 6 à 10 atomes de carbone et un polyglycérol ayant un degré moyen de polymérisation de 3 ou plus et de moins de 100, un agent tensioactif non ionique, et un agent huileux ayant un point de fusion de 25°C ou moins, ou ayant une fluidité dans lequel des substances solides sont dispersées dans une substance liquide ayant un point de fusion de moins de 25°C, dans laquelle l'agent huileux est présent dans une quantité de 70 à 98% en poids sur la base de la composition contenant un ester d'acide gras à chaîne moyenne de polyglycérol.

2. Utilisation selon la revendication 1, dans laquelle l'agent huileux est choisi parmi des graisses et des huiles naturelles animales ou végétales et des graisses et des huiles semi-synthétiques, des huiles hydrocarbures, des huiles d'ester, des huiles de glycéride, des huiles de silicone, des vitamines solubles dans la graisse, des acides gras supérieurs, et des composants d'huiles purifiées provenant d'animaux et de plantes ou des huiles synthétiques.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0582246 A2
- JP 8099852 A
- JP 2003012456 A
- JP 2001025654 A
- JP 2003238396 A
- JP 2004067587 A
- JP 2004277375 A
- JP 2003040708 A
- EP 0334777 A1

**Non-patent literature cited in the description**

- **J. H. SCHULMAN ; W. STOECKENIUS ; L. M. PRINCE.** *J. Phys. Chem.,* 1959, vol. 63, 1677